# EUROPÄISCHE PATENTANMELDUNG

(11) **EP 0 701 987 A1**
(43) Veröffentlichungstag der Anmeldung: **20.03.1996**
(21) Anmeldenummer: 94810575.4
(22) Anmeldetag: 03.10.1994
(51) Int. Cl.: C07C 45/46, C07C 49/76, C07C 49/788

(54) **Verfahren zur Herstellung von aromatischen Ketonen**

(30) Priorität: 16.09.1994 CH 2837/94
(71) Anmelder: CU CHEMIE UETIKON AG, CH-8707 Uetikon am See (CH)
(72) Erfinder: Vogt, Andreas, Dr., CH-8032 Zürich (CH); Pfenninger, Armin, Dr., CH-8707 Uetikon (CH)
(74) Vertreter: Werffeli, Heinz R., Dipl.-Ing.ETH.

(57) **Zusammenfassung**

Zur Herstellung von aromatischen Ketonen mit der allgemeinen Formel 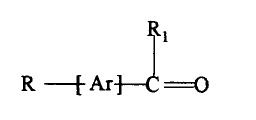
worin R für Methyl, Ethyl, Isopropyl, Isobutyl, geradkettiges und verzweigtes C₃ - C₁₄-Alkyl, Ar gleich für Phenyl- oder Naphthyl, und R₁ für Methyl, Ethyl, geradkettiges oder verzweigtes C₃ - C₆-Alkyl stehen kann, werden alkylsubstituierte Benzole mit Carbonsäureanhydriden, Acylchloriden oder freien Carbonsäuren bei einer Temperatur im Bereich von 50 - 250°C an einem sich in der aciden Form befindlichen siliciumreichen Zeolithen umgesetzt.

## Beschreibung

Die Erfindung betrifft ein Verfahren zur Herstellung von aromatischen Ketonen gemäss dem Oberbegriff des Patentanspruchs 1.

Die Acylierung von aromatischen Substraten ist eine nützliche Methode zur Herstellung von Ausgangs- oder Zwischenprodukten in der pharmazeutischen Industrie, wie sie etwa zur Gewinnung der 2-(4'-Isobutylphenyl)-propionsäure (Ibuprofen) verwendet werden.

Ibuprofen ist ein bekanntes, nicht-steroides Schmerzmittel welches generell über die Acetylierung von Isobutylbenzol (IBB) zu p-Isobutylacetophenon (IBAP) hergestellt wird. Die Reduktion durch katalytische Hydrierung von IBAP führt zu 1-(4'-Isobutylphenyl)ethanol (IBPE), Carbonylierung von IBPE resultiert schlussendlich in Ibuprofen (EP-A-0 284 310).

Es ist bekannt, dass p-Isobutylacetophenon durch Friedel-Crafts-Acylierung von Isobutylbenzol mittels geeigneter Acetylierungsreagenzien und wasserfreiem Aluminiumchlorid in ver-schiedenen Lösungsmitteln, wie vorzugsweise Schwefelkohlenstoff oder Methylenchlorid, gewonnen werden kann. Diese Verfahren liefern das p-Isobutylacetophenon jedoch nur in mässiger Reinheit und ungünstigen Ausbeuten, was oft durch die Bildung eines beträchtlichen Anteils an Nebenprodukten bedingt ist.

Die Synthese von p-Isobutylacetophenon (IBAP) aus Isobutylbenzol wird in der Literatur beschrieben. Zum Beispiel haben Bradford et al. im Journal of the American Chemical Society, 4943 - 4945 (1956) auf Seite 4945 die Synthese von p-Isobutylacetophenon (IBAP) mittels einer Friedel-Crafts-Acylierung von Isobutylbenzol mit Essigsäureanhydrid in Gegenwart von Aluminiumchlorid als Katalysator beschrieben.

Die EP-A-400,892 beschreibt die Produktion von Isobutylacetophenon mittels Essigsäureanhydrid in Gegenwart von HF als Katalysator und Lösungsmittel.

Zur Zeit erfolgt die industrielle Acylierung von Alkylaromaten allgemein mittels Säurechloriden als Acylierungsagens zusammen mit stöchlometrischen Mengen an Protonsäuren wie HF, H₂SO₄ und HCl oder mit Metallhalogeniden wie AlCl₃, TiCl₄, FeCl₃ und BF₃. In dieser Art von Syntheseprozess fallen grosse Abfallmengen an. Die teilweise grösser als stöchiometrischen Mengen an Katalysator werden zur Komplexierung des Ketons gebraucht. Der anschliessende Hydrolyseschritt verursacht die Zerstörung des Katalysators, die Freisetzung grosser Mengen an Zersetzungsgasen, was zu grossen Korrosionsproblemen führt.

Aufgabe der vorliegenden Erfindung ist die Schaffung eines Verfahrens der eingangs genannten Art, bei welchem ein regenerierbarer und in geringerer Menge erforderlicher Katalysator verwendet werden kann.

Diese Aufgabe wird erfindungsgemäss mittels eines Verfahrens nach Patentanspruch 1 gelöst, d.h. durch die Acylierung von aromatischen Reaktanden mit Säureanhydriden oder Acylhalogeniden an sauren Zeolith-Katalysatoren. Dieses Verfahren ist vor allem nützlich für die hoch selektive para-Acylierung von monosubstituiertem Benzol.

Zweckmässige Weiterausgestaltungen des erfindungsgemässen Verfahrens sind Gegenstand der Ansprüche 2 bis 9.

Der Gebrauch von Zeolithen als Katalysatoren für die Friedel-Crafts-Acylierung löst viele der bisherigen Probleme. Zeolithe sind thermisch äusserst stabil und können durch einen einfachen Kalzinierungsschritt zur Entfernung kokshaltiger Ablagerungen regeneriert bzw. neu aktiviert werden. Ausserdem sind nur wirklich katalytische Mengen (max. 10 % vom Substratgemisch) notwendig, um die Acetylierungsreaktion zu katalysieren. Die Separierung des Katalysators vom Reaktionsgemisch kann über eine einfache Filtration erfolgen.

Gemäss dieser Erfindung wird ein fester, saurer Katalysator (Zeolith) verwendet und ein Verfahren zur Anwendung eines solchen Katalysators in der heterogen katalysierten Acylierung von aromatischen Substraten mit Acylierungsmitteln, die ein relativ niedriges Molekulargewicht besitzen, beschrieben. Obwohl der Katalysator bevorzugt unter milden Temperaturbedingungen, in der flüssigen Phase, benutzt wird, ist auch ein Gebrauch unter Gasphasenbedingungen möglich. Das Produkt wird in einer hoch selektiven Acylierungsreaktion gewonnen, was eine Reinigung des Endproduktes von entstandenen Nebenprodukten überflüssig macht. Diese Acylierungsreaktion wird durchgeführt über das Zusammenbringen von Alkylbenzol und einem geeigneten Acylierungsreagens in der Gegenwart von katalytischen Mengen eines hoch kristallinen, siliciumreichen Zeolithen (z.B. Beta, USY, ZSM-5), wobei der Zeolith sich in der aciden H-Form befindet. Die Reaktion wird bei einer Temperatur von 50 - 250 °C, vorzugsweise 100 - 200°C durchgeführt.

Nachstehend wird die Erfindung anhand einer Anwendung in einem Syntheseprozess beispielsweise beschrieben.

Der Katalysator besteht dabei aus einem siliciumreichen Zeolithen in der aciden H-Form, welcher vor allem für die Acylierung von aromatischen Substraten mittels einem Acylierungs-Agens, welches ein niedriges Molekulargewicht besitzt, geeignet ist. Diese Erfindung wird dabei in der Acetylierung von Isobutylbenzol, bei der unter milden Flüssigphasen-Bedingungen selektiv p-Isobutylacetophenon produziert werden kann, angewendet.

Die Acylierung verläuft nach folgender Gleichung: 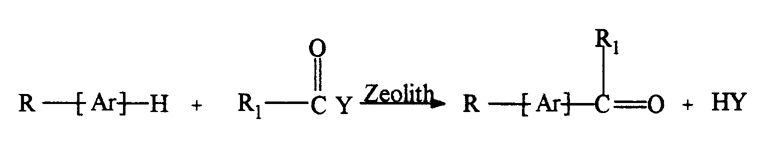
worin R für Methyl, Ethyl, Isopropyl, Isobutyl, geradkettiges und verzweigtes C₃ - C₁₄ Alkyl steht und Ar gleich Phenyl- oder Naphthyl- ist, R₁ ist Methyl, Ethyl, geradkettiges oder verzweigtes C₃ - C₆-Alkyl und Y kann eine Gruppe bestehend aus Hydroxy-, Acyloxy- oder Halogen sein.

Es wurde dabei gefunden, dass bei der Acetylierung von Isobutylbenzol mittels ausgewählter Zeolithkatalysatoren eine höhere Selektivität erreicht wird als beim klassischen Friedel-Crafts-System. Zudem treten keinerlei Abfallprobleme bezüglich des Katalysators auf, da dieser nach einer entsprechenden Reaktivierung erneut in das System zurückgeführt werden kann. Solche ausgewählten Katalysatoren sollen nun im Detail beschrieben werden.

Dieses Verfahren ist vorteilhafterweise durch die Benutzung eines Zeolithen vom Beta-Typ als Katalysator gekennzeichnet.

Zeolithe sind durch die allgemeine Formel (I) charakterisiert:

M¹ₘ[mM²O₂·nSiO₂]·qH₂O (I)

Hierin entspricht M¹ einem Aequivalent eines austauschbaren Kations, dessen Anzahl m dem Anteil von M² entspricht;
M² einem dreiwertigen Element, welches gemeinsam mit dem Si das oxydische Gerüst des Zeolithen bildet;
n/m dem SiO₂/M²O₂-Verhältnis;
q der Menge des adsorbierten Wassers.
Zeolithe sind von ihrer Grundstruktur her kristalline Alumosilikate, die aus einem Netzwerk von SiO₄- bzw. M²O₄-Tetraedern aufgebaut sind. Die einzelnen Tetraeder sind mit Sauerstoffbrücken über die Ecken der Tetraeder untereinander verknüpft und bilden ein räumliches Netzwerk, das gleichmässig von Kanälen und Hohlräumen durchzogen ist. Die einzelnen Zeolithstrukturen unterscheiden sich durch ihre Anordnung und Grösse der Kanäle und Hohlräume sowie durch ihre Zusammensetzung. Als Ausgleich für die negative Ladung des Gitters, die durch den Anteil an M² zustande kommt, sind austauschbare Kationen eingelagert. Die adsorbierte Wasserphase q H₂O ist reversibel entfernbar, ohne dass das Gerüst seine Struktur verliert.

M² ist vielfach Aluminium, kann aber durch andere dreiwertige Elemente teilweise oder ganz substituiert sein.

Die erfindungsgemäss einsetzbaren Zeolithtypen gehören zur Gruppe der sogenannten mittel- und grossporigen Zeolithe mit Porenweiten ≧ 5 Å.

Bei der Herstellung von IBAP werden bevorzugt sind solche Zeolithe des Beta-Typs verwendet, bei denen mindestens ein Teil der Metallkationen gegen Wasserstoffionen ausgetauscht worden sind, vorzugsweise 50 - 100 %, besonders bevorzugt 80 - 100 % aller ursprünglich vorhandenen Metallkationen. Die sauren H⁺-Formen der Zeolithe werden beispielsweise dadurch hergestellt, dass man Metallionen gegen Ammoniumionen austauscht und den so ausgetauschten Zeolithen anschliessend kalziniert. Dieser 2-Schritt-Austausch-Vorgang lässt sich auch mit verdünnten Mineralsäuren in einem 1-Schritt-Austauschprozess durchführen.

Während das in Formel (I) definierte SiO₂/M²O₂-Verhältnis n/m bei siliciumreichen Zeolithen Werte von 10 - 200 : 1 und grösser annehmen kann, werden für das vorliegende Verfahren beispielsweise Zeolithe vom Beta-Typ eingesetzt, bei denen n/m Werte von 5 - 100 : 1 annimmt. Begünstigt werden n/m Werte von 10 - 80 : 1.

Die Einsatzprodukte können gasförmig, aber auch ganz oder teilweise flüssig mit dem Katalysator in Kontakt gebracht werden. Von Vorteil ist es jedoch, wenn sowohl das Isobutylbenzol alsauch das Essigsäureanhydrid bzw. Acetylchlorid flüssig an den aktivierten Katalysator herangebracht werden. Hierzu wählt man eine Temperatur im Bereich von 50 bis 250 °C.

Bevorzugt wird die Reaktion bei Atmosphärendruck durchgeführt, was auch eine verfahrenstechnisch einfache Reaktionsführung gewährleistet.

Bei der bevorzugten Durchführungsform wird das Reaktionsgemisch Isobutylbenzol/Essigsäureanhydrid (oder Acetylchlorid) in einen Batch-Reaktor eingebracht und bei Raumtemperatur gerührt. Der frisch aktivierte Zeolith-Katalysator wird gegebenenfalls unter einem leichten Inertgasstrom in das Reaktionsgemisch eingetragen. Eine besonders begünstigte Temperatur für diese Flüssigphasenreaktion ist hierbei der Bereich von 100 - 200 °C.

Zur Aufarbeitung kann das gewünschte Produkt p-Isobutylacetophenon destillativ vom Reaktionsgemisch getrennt und gereinigt werden. Nicht umgesetztes Isobutylbenzol und nicht umgesetztes Essigsäurenahydrid bzw. Acetylchlorid können in bekannter Weise in die Reaktion zurückgeführt werden.

### Beispiel

In einen 100 ml Reaktor werden 13.4 g (0.1 mol) Isobutylbenzol (IBB) und 1.02 g (0.01 mol) Essigsäureanhydrid (Ac₂O) vorgelegt. Unter Rühren wird 1g frisch aktivierter Zeolith H-Beta unter Rühren bei einer Reaktionstemperatur von 140 °C in die Reaktionlösung überführt. Nach 1, 2 und 8 Stunden Reaktionszeit werden jeweils Proben entnommen und gaschromatographisch analysiert.

Man erhielt folgende Ergebnisse:

| Katalysator SiO₂/Al₂O₃ | Temperatur [° C] | Zeit[Std.] | Ausbeute [mol %] * | Selektivität [mol %] ** |
|---|---|---|---|---|
| H-Beta 23 | 140 | 1.0 | 80.7 | 96.5 |
| H-Beta 23 | 140 | 2.0 | 83.9 | 95.7 |
| H-Beta 23 | 140 | 8.0 | 86.9 | 95.0 |

| | | | | |
|---|---|---|---|---|
| * mol % p-Isobutylacetophenon (IBAP) bezogen auf Ac₂O | | | | |
| ** 100 x Ausbeute IBAP/Umsatz Ac₂O | | | | |

## Patentansprüche

1. Verfahren zur Herstellung von aromatischen Ketonen mit der allgemeinen Formel 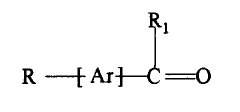 worin R für Methyl, Ethyl, Isopropyl, Isobutyl, geradkettiges und verzweigtes C₃ - C₁₄-Alkyl, Ar gleich für Phenyl- oder Naphthyl, und R₁ für Methyl, Ethyl, geradkettiges oder verzweigtes C₃ - C₆ -Alkyl stehen kann, gekennzeichnet durch Umsetzung von alkylsubstituierten Benzolen mit Carbonsäureanhydriden, Acylchloriden oder freien Carbonsäuren bei einer Temperatur im Bereich von 50 - 250 °C an einem sich in der aciden Form befindlichen siliciumreichen Zeolithen mit einer Porenweite ≧ 5 Å, mit einem SiO₂/M²O₂ Verhältnis von 5 - 200 : 1, wobei M² ein Element oder mehrere aus der Gruppe Al, B, Ga, In, Fe, Cr, V, As, und Sb in dreiwertiger Form darstellt.

2. Verfahren nach Anspruch 1, dadurch gekennzeichnet, dass das Molverhältnis Alkylbenzol/ Acylierungsagens 1 - 30 : 1, vorzugsweise 8 - 20 : 1 beträgt.

3. Verfahren nach Anspruch 1, dadurch gekennzeichnet, dass die Umsetzung bei einer Temperatur im Bereich von 100 - 200°C durchgeführt wird.

4. Verfahren nach Anspruch 1 dadurch gekennzeichnet, dass Isobutylbenzol mit Essigsäure- anhydrid in der Flüssigphase zu p-Isobutylacetophenon umgesetzt wird, wobei ein Produkt mit gesteuerter Selektivität entsteht.

5. Verfahren nach Anspruch 1 dadurch gekennzeichnet, dass es sich beim Katalysator um einen Beta-, USY oder ZSM-5-Zeolithen handelt.

6. Verfahren nach Anspruch 5, dadurch gekennzeichnet, dass man Zeolithe des Beta-Typs verwendet, bei denen mindestens ein Teil der Metallkationen gegen Wasserstoffionen ausgetauscht worden ist.

7. Verfahren nach Anspruch 6, dadurch gekennzeichnet, das 50 - 100 %, vorzugsweise 80 - 100 % aller ursprünglich vorhandener Metallkationen der Zeolithe gegen Wasserstoffionen ausgetauscht worden ist.

8. Verfahren nach Anspruch 5, dadurch gekennzeichnet, dass man Zeolithe des Beta-Typs verwendet, bei denen das SiO₂/M²O₂ - Verhältnis 5 - 100 : 1, vorzugsweise 10 - 80 : 1, beträgt.

9. Verfahren nach den Ansprüchen 4 und 5, dadurch gekennzeichnet, dass die Flüssig-phasenreaktion bei einer Temperatur im Bereich von 100 - 200 °C durchgeführt wird.

10. Anwendung des Verfahrens nach Anspruch 1 zur Herstellung von p-Isobutylacetophenon.
